# EUROPEAN PATENT SPECIFICATION

(11) **EP 2 020 925 B1**
(45) Date of publication and mention of the grant of the patent: **10.07.2013**
(21) Application number: 07777322.4
(22) Date of filing: 30.05.2007
(51) Int. Cl.: A61B 17/122, A61B 17/128, A61B 17/064, A61B 19/00

(54) **RELEASE MECHANISMS FOR A CLIP DEVICE**
LÖSEMECHANISMUS FÜR EINE KLAMMERVORRICHTUNG
MÉCANISMES DE LIBÉRATION D'UN DISPOSITIF D'AGRAFE

(30) Priority: 01.06.2006 US 809912 P
(43) Date of publication of application: 11.02.2009
(73) Proprietor: Cook Medical Technologies LLC, Bloomington, IN 47404 (US)
(72) Inventor: BROWN, Hilbert, D., Winston Salem, NC 27101 (US); CHEN, Steve, K., Lafayette, IN 47905 (US); GAYZIK, Caroline, M., Winston Salem, NC 27104 (US); DUCHARME, Richard, W., Winston Salem, NC 27106 (US); KARPIEL, John, A., Winston Salem, NC 27106 (US); KORNRUMPF, Kathryn, M., Winston Salem, NC 27103 (US); SURTI, Vihar, C., Winston Salem, NC 27106 (US)
(74) Representative: Garratt, Peter Douglas
(86) International application number: PCT/US2007/012754
(87) International publication number: WO 2007/142977

(56) References cited:
- EP-A- 1 604 614
- WO-A-03/030746
- DE-C1- 19 534 320
- US-A1- 2004 092 978

## Description

### TECHNICAL FIELD

The present invention relates to a clip, and more specifically, to a clip that can be used to cause hemostasis of blood vessels along the gastrointestinal tract, or that can be used as an endoscopic tool for holding tissue or the like.

### BACKGROUND INFORMATION

Conventionally, a clip may be introduced into a body cavity through an endoscope to grasp living tissue of a body cavity for hemostasis, marking, and/or ligating. In addition, clips are now being used in a number of applications related to gastrointestinal bleeding such as peptic ulcers, Mallory-Weiss tears, Dieulafoy's lesions, angiomas, post-papillotomy bleeding, and small varices with active bleeding.

Gastrointestinal bleeding is a somewhat common and serious condition that is often fatal if left untreated. This problem has prompted the development of a number of endoscopic therapeutic approaches to achieve hemostasis such as the injection of sclerosing agents and contact thermo-coagulation techniques. Although such approaches are often effective, bleeding continues for many patients and corrective surgery therefore becomes necessary. Because surgery is an invasive technique that is associated with a high morbidity rate and many other undesirable side effects, there exists a need for highly effective, less invasive procedures.

Mechanical hemostatic devices have been used in various parts of the body, including gastrointestinal applications. Such devices are typically in the form of clamps, clips, staples, sutures, etc. that are able to apply sufficient constrictive forces to blood vessels so as to limit or interrupt blood flow. One of the problems associated with conventional hemostatic devices, however, is that they can only be delivered using rigid shafted instruments via incision or trocar cannula. Moreover, many of the conventional hemostatic devices are not strong enough to cause permanent hemostasis.

One proposed solution is described in U.S. Pat. No. 5,766,189, which shows a clip device having a pair of arms that are provided with a tendency to open. One problem with this clip and other similar types of clips having a pair of arms is that it may often be necessary to rotate the clip to properly grasp the area to be clipped. Rotation of the clip is often hindered or complicated by the travel of the operating wire through the bends of the tube(s) used to deliver the clip. Accordingly, there is a need for a clip that can be delivered to the target area and used without having to rotate the clip to a desired orientation.
Reference is also directed to DE 195 34 320 in which two elastic and flexible arms are held at one end, and are enclosed by a locking ring sliding between the open and shut positions. There is a forceps clamping the clip at one end, while a lengthwise-sliding tube encloses the forceps in one end position, securing it in the end of the instrument. There is a face on one end of it acting against the locking ring so as to thrust this onto the arms of the clip. At its free end, the tube forms an annular forceps accommodating the locking ring. It is in turn enclosed by an outer tube sliding on it between a position shutting the forceps and one releasing it. Reference is directed to EP 1 604 614 which describes a clip with a securing ring that can be fixed in a recess port

Another problem often encountered with conventional hemostatic devices is the difficulty in securing the clip device to the delivery apparatus prior to reaching the target area within the patient, and then quickly and easily releasing the clip device from the delivery apparatus once the clip has been attached to the target site.

Therefore, there is a need for a release mechanism that may quickly and reliably disengage the clip device from the delivery apparatus once the clip has been attached to the target site.

### SUMMARY

A clip device for living tissue in a body cavity according to the present invention comprises an outer sheath that is insertable into the body cavity. Disposed within the outer sheath is an inner sheath. The inner sheath is independently slidable within the outer sheath.
A clip is provided with a proximal end from which at least two arms extend. The arms are formed of a resilient material and are shaped such that the arms are biased or have a tendency to be in an open position.

In a first embodiment, a first retainer is attached to the proximal end of the clip. An operating wire is slidably disposed within an inner portion of the inner sheath, and has a distal end portion with a second retainer attached to the distal end thereof. The second retainer releasably mates with the first retainer to couple the clip to the operating wire. A sliding ring is provided and is configured such that when the sliding ring is moved over the arms it holds them in a closed position. The sliding ring has a portion that is sized to contact the inner sheath so that when the inner sheath is advanced, the sliding ring slides over the arms of the clip to close them.

In one method of operation, the two retainers are joined together and the sliding ring is moved to a position such that the sliding ring covers the two retainers. As a result, the clip is joined with the operating wire. The outer sheath is advanced to a position over the clip to compress or collapse the arms within the device so that it may be passed into a channel of an endoscope. When the device is at the target site, the outer sheath is retracted to expose the arms, causing them to open radially outward. The inner sheath is advanced, pushing the sliding ring over the arms so as to close the arms onto the tissue. Thereafter, when the inner sheath is retracted, the retainers may be released, the device is retracted, and the clip and first retainer are left behind.

Optionally, stop elements, such as beads, may be disposed on the clip to ensure that the sliding ring is not advanced distally beyond the end of the clip. Further, the stop elements may lockingly engage with the sliding ring to ensure that the sliding ring does not disengage from the clip.

In alternative embodiments, the first retainer may be disengaged from the second retainer, for example, by retracting the second retainer with respect to the first retainer, rotating the second retainer with respect to the first retainer, or simply removing the sliding ring or inner sheath so that they no longer radially restrain the retainers.

In a further alternative embodiment, an alternative clip is disclosed comprising at least two arms having substantially flat regions along part or all of their length. The proximal ends of the arms unite at the proximal end of the clip. The proximal end of the clip has a hole formed therein. Various means are disclosed for coupling an operating wire to the clip using the hole at the proximal end of the clip.

Other systems, features and advantages of the invention will be, or will become, apparent to one with skill in the art upon examination of the following figures and detailed description. It is intended that all such additional systems, features and advantages be within the scope of the invention, and be encompassed by the following claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.

FIG. 1 is an illustration of one embodiment of a clip device according to the present invention.

FIG. 2 is a partial side-sectional view of a portion of the clip device of FIG. 1 before the retainers are joined.

FIG. 3A is a side-sectional view of a portion of the clip device of FIG. 1 after the retainers are joined.

FIG. 3B is a side-sectional view of an alternative clip device of FIGS. 1-3A.

FIG. 3C is a side-sectional view of a further alternative clip device of FIGS. 1-3A.

FIG. 4 is a side-sectional view of an alternative release mechanism that may be used to deploy a clip device.

FIGS. 5A-5C are, respectively, a side-sectional view of an alternative release mechanism that may be used to deploy a clip device, a side-sectional view of the first retainer of FIG. 5A after deployment, and a side-sectional view of a further alternative release mechanism.

FIG. 6 is a side view of an alternative release mechanism that may be used to deploy a clip device.

FIGS. 7A-7B are, respectively, a side-sectional view of an alternative release mechanism that may be used to deploy a clip device, and an end view showing the distal end of the sliding ring of FIG. 7A.

FIG. 8 is a side-sectional view of an alternative release mechanism that may be used to deploy a clip device.

FIGS. 9A-9B are side-sectional views of alternative release mechanisms that may be used to deploy a clip device.

FIGS. 10A-10B are side-sectional views of alternative release mechanisms that may be used to deploy a clip device.

FIGS. 11A-11B are, respectively, a side-sectional view of an alternative release mechanism that may be used to deploy a clip device, and a side view of the inner sheath and sliding ring of FIG. 11A.

FIG. 12 is a side view of an alternative release mechanism that may be used to deploy a clip device.

FIGS. 13A-13B are, respectively, a side view and a top view of an alternative clip of the present invention.

FIG. 14 is a side-sectional view illustrating a method of deploying the clip of FIGS. 13A-13B.

FIG. 15 is a side-sectional view illustrating an alternative method of deploying the clip of FIGS. 13A-13B.

FIG. 16 is a side-sectional view illustrating an alternative method of deploying the clip of FIGS. 13A-13B.

FIG. 17 is a side-sectional view illustrating an alternative method of deploying the clip of FIGS. 13A-13B.

FIGS. 18A-18C are side-sectional views illustrating an alternative retainer system.

FIG. 19 is a side-sectional view illustrating a clip retaining apparatus.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patent's anatomy during a medical procedure.

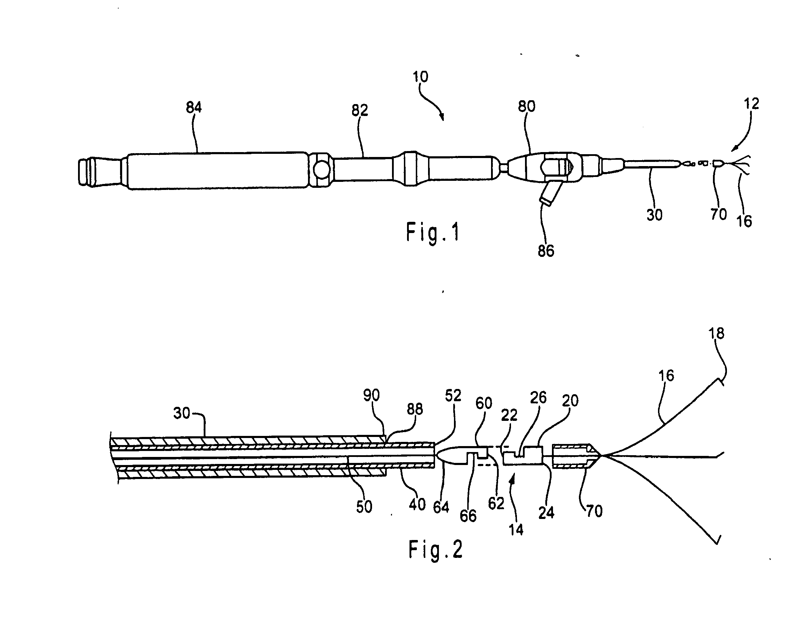

The present invention provides a clip device for tissue or the like. Referring to FIGS. 1-3A, a first embodiment of a clip device according to the present invention is shown. Clip device 10 includes clip 12 with proximal end 14 having three arms 16 extending from the proximal end. Each arm is preferably inwardly bent at its end 18 to better grasp the tissue. While three arms are preferred, it is contemplated that fewer than or more than three arms may be used. For example, clip 12 may have two or four arms.

The clip may be made from any suitable resilient material such as stainless steel, nitinol, plastic, and the like. In addition, the arms may have a cross-sectional shape that is round, square, triangular, pie-shaped, truncated cone, and the like.

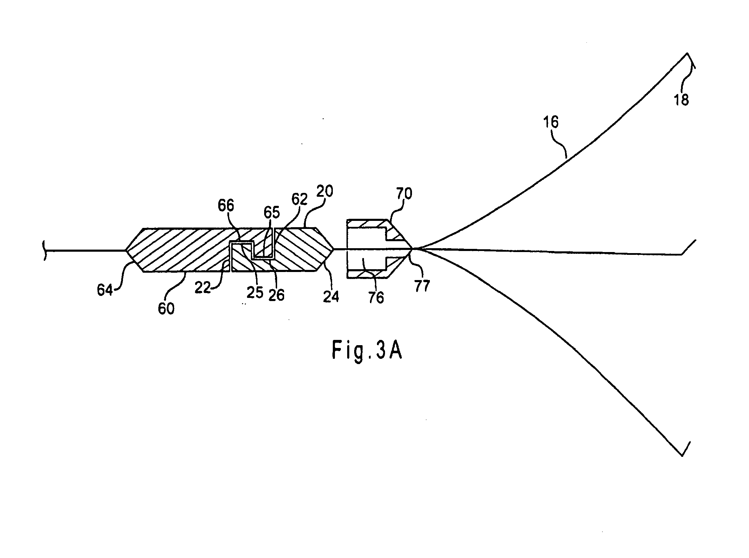

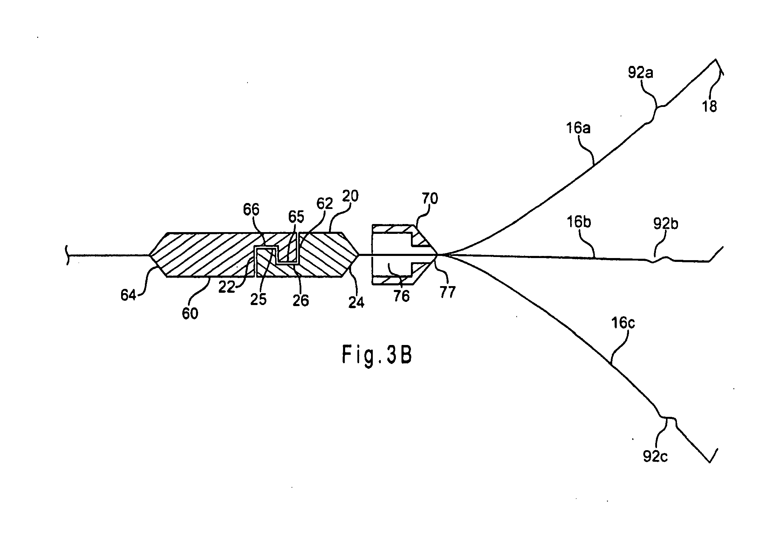

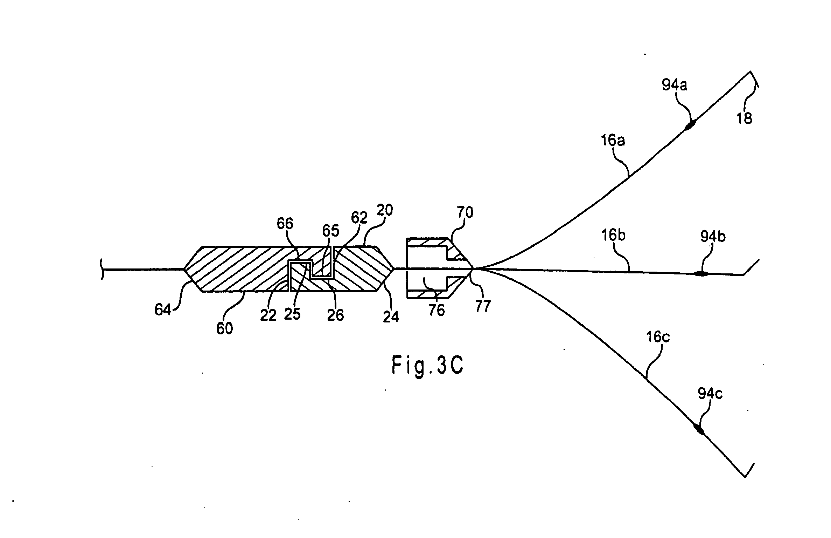

The proximal end 14 of the clip comprises first retainer 20 attached to the arms. In one embodiment, the first retainer is permanently attached to the arms. The retainer preferably is provided with a shape that will complement a shape provided on a second retainer so that the first and second retainers will matingly join with each other. For example, in the embodiment of FIGS. 1-3A, first retainer 20 has proximal end 22 and distal end 24, with notch 26 being disposed therebetween. In this embodiment, proximal end 22 approximates the shape of a half-cylinder having a flat top surface 25, as depicted in FIG. 3. As will be explained in more detail below, this shape advantageously provides secure mating with complementary second retainer 60 without increasing the diameter beyond that of the first end of the retainer.

Clip device 10 also comprises outer sheath 30 (or an introducing tube) having an inner diameter that receives inner sheath 40. The inner sheath can be advanced and retracted independently of the outer sheath. Inner sheath 40 has an inner diameter that receives operating wire 50.

Outer sheath 30 is attached at its proximal end to forward handle portion 80. Inner sheath 40 extends through forward handle portion 80 and is attached at its proximal end to middle handle portion 82, which is disposed proximally of the forward handle portion. Operating wire 50 extends through the forward and middle handle portions, and is attached at its proximal end to rearward handle portion 84, which telescopically extends over the proximal portion of the middle handle portion. As will be explained in more detail below, longitudinal movement of the operating wire and the inner and outer sheaths with respect to each other is controlled by longitudinal manipulation of the forward, middle and rearward handles portions with respect to each other.

Forward handle portion 80 preferably includes flushing port 86. The flushing port may comprise a standard male or female luer fitting, or any other valve mechanism that permits the injection of fluid therethrough. The flushing port is in fluid communication with an interior volume of forward handle portion 80, which in turn is in fluid communication with a cavity or gap 88 that is disposed between the inner and outer sheaths. Accordingly, any fluid injected through flushing port 86 will necessarily enter cavity 88 between the inner and outer sheaths, and will subsequently exit cavity 88 near distal end 90 of outer sheath 30 (see FIG. 2). In other words, the fluid injected through the flushing port will exit the clip device near the clip.

Alternatively, the cavity can be disposed inside inner sheath 40, or either the inner or the outer sheath may comprise a lumen disposed therein through which fluid can be passed along the length thereof. It should also be understood that the flushing port could be alternatively located on either of the middle or rearward handle portions, or on a portion of the outer sheath distally of any of the handle portions.

In the embodiment of FIGS. 1-3A, second retainer 60 is attached to the distal end of operating wire 50. Preferably, second retainer 60 is complementary to first retainer 20 so that the first and second retainers can be matingly joined. Accordingly, second retainer 60 has proximal end 64 and distal end 62, with notch 66 being disposed therebetween. In this embodiment, distal end 62 approximates the shape of a half-cylinder having a flat surface 65, as depicted in FIG. 3A.

The first and second retainers are joined with each other by locating flat surface 25 of first retainer 20 within notch 66 of second retainer 60, and by locating flat surface 65 of second retainer 60 within notch 26 of first retainer 20. When joined, the first and second retainers form a substantially continuous cylinder shape having substantially the same outer diameter from proximal end 64 of second retainer 60 to distal end 24 of first retainer 20, as shown in FIG. 3A.

It will be understood by one of skill in the art that, although first retainer 20 matingly joins with second retainer 60, they will not retain a joined position unless they are held together. Accordingly, sliding ring 70 is provided and has a first inner diameter 76 slightly larger than an outer diameter of first retainer 20 and second retainer 60. In other words, the first inner diameter 76 of sliding ring 70 is such that the sliding ring can slide over the retainers, yet hold and maintain them in a mating position. In addition, sliding ring 70 can slide toward the ends of arms 16 of clip 12, causing the arms to move to a closed position, as explained below.

One possible method of operation of the first embodiment will be described. Outside of the patient's body, outer sheath 30 is retracted to expose inner sheath 40, operating wire 50, and second retainer 60. Clip 12 is provided and first retainer 20 is matingly joined with second retainer 60, as described with respect to FIG. 3A above. Sliding ring 70 is placed over first retainer 20 and second retainer 60 so that they are maintained in a joined position. Sliding ring 70, having the retainers secured therein, then is disposed distal to inner sheath 40 and within outer sheath 30.

In a next step, outer sheath 30 is pushed toward the distal end of inner sheath 40 and beyond the clip, causing the arms of the clip to close. In this state, outer sheath 30 is introduced into a body cavity via a working channel of an endoscope (not shown) that has been previously inserted into the body cavity. While the body cavity is observed via the endoscope, the distal end portion of outer sheath 30 is guided to a part to be treated.

If the part to be treated is obscured by blood or other bodily fluids, then a fluid such as saline is injected through flushing port 86 on forward handle portion 80. The fluid enters the cavity or gap between inner sheath 40 and outer sheath 30, and exits the distal end of the outer sheath. The fluid floods the area so as to flush any blood or bodily fluids away from the part to be treated. The injection of fluid is continued and/or repeated as necessary during the following steps so as to keep the area free of blood and other bodily fluids.

Alternatively, a vacuum force may be applied to flushing port 86 so as to create suction within the cavity or gap between the inner and outer sheaths. This suction can be used to remove blood or other bodily fluids from the area surrounding the part to be treated.

In a next step, outer sheath 30 is retracted proximally to expose clip 12, which causes arms 16 to extend in a radially outward direction, as generally depicted. Inner sheath 40 is then advanced towards clip 12, causing sliding ring 70 to slide toward arms 16 of clip 12 and causing the arms to close, thereby grasping the tissue and facilitating tissue closure. Inner sheath 40 is then retracted and when the distal end of the inner sheath passes the first and second retainers, they detach and release from each other. Clip 12 is left inside the body cavity, holding the tissue. After disengaging the retainers, the clip operating device is removed from the channel of the endoscope.

In the embodiment illustrated, the distal opening 77 of sliding ring 70 has a second inner diameter smaller than a first diameter on first retainer 20. As a result, the sliding ring is not removable from the clip. In this embodiment, the sliding ring can be located adjacent the proximal end of the clip so that the arms are in an open position. The sliding ring can then be moved to a position toward the ends of the arms to close them.

Referring now to FIGS. 3B-3C, alternative embodiments of the clip device of FIGS. 1-3A are described. In FIG. 3B, the three arms 16a-16c of clip 12 comprise kinks 92a, 92b and 92c, respectively, which may be formed by bending or warping portions of the arms as depicted. The distal opening 77 of sliding ring 70 has a second inner diameter configured to frictionally engage kinks 92a-92c of the three arms 16a-16c. In use, sliding ring 70 slides toward the ends of arms 16a-16c of clip 12, causing the arms to move to a closed position, as explained above. Once distal opening 77 of sliding ring 70 engages kinks 92a-92c of arms 16a-16c, respectively, kinks 92a-92c preferably become wedged within distal opening 77 and limit further distal movement of sliding ring 70. In effect, kinks 92a-92c serve as distal stop elements to ensure that the sliding ring cannot pass distally over the clip.

In FIG. 3C, the three arms 16a-16c of clip 12 comprise increased diameter portions 94a, 94b and 94c, respectively. Increased diameter portions 94a-94c may have diameters slightly greater than remaining portions of arms 16a-16c. The distal opening 77 of sliding ring 70 has a second inner diameter configured to frictionally engage increased diameter portions 94a-94c of the three arms 16a-16c. In use, sliding ring 70 slides toward the ends of arms 16a-16c of clip 12, causing the arms to move to a closed position, as explained above. Once distal opening 77 of sliding ring 70 engages increased diameter portions 94a-94c of arms 16a-16c, respectively, the increased diameter portions 94a-94c preferably become wedged within distal opening 77 and limit further distal movement of sliding ring 70 to ensure that the sliding ring cannot pass distally over the clip.

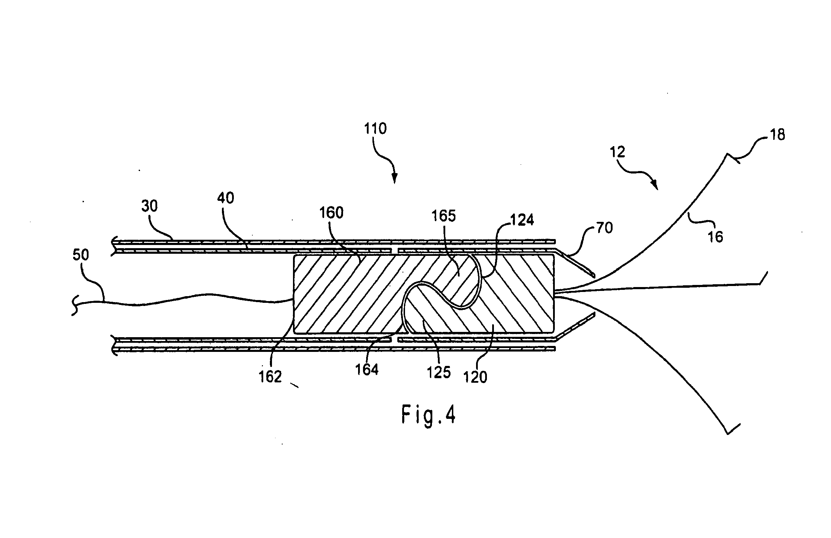

Referring now to FIGS. 4-12, various alternative release mechanisms for deploying a clip device are described. In general, the release mechanisms described in FIGS. 4-12 may be used in conjunction with apparatus described in FIGS. 1-3. For example, outer sheath 30, inner sheath 40, operating wire 50, sliding ring 70, forward handle portion 80, middle handle portion 82, rearward handle portion 84 and flushing port 86 may be used in the embodiments of FIGS. 4-12. Further, clip 12 may be provided in accordance with the embodiments described above, e.g., comprising three arms 16 and preferably having an inward bend 18 at its distal end to facilitate hemostasis.

Referring to FIG. 4, a first alternative embodiment for deploying clip 12 is provided. Alternative clip device 110 comprises first retainer 120 and second retainer 160. First retainer 120 is operably attached to arms 16 of clip 12. Proximal end 162 of second retainer 160 is attached to operating wire 50, as shown in FIG. 4. First retainer 120 and second retainer 160 preferably are cylindrical in cross-sectional shape and have substantially identical outer diameters when mating, as described below.

First retainer 120 comprises partially rounded notch 124 formed therein, and has rounded knob 125 formed proximal to notch 124. Similarly, second retainer 160 comprises partially rounded notch 164 formed therein, and has rounded knob 165 disposed distal to notch 164. During delivery of the device, rounded knob 165 is aligned with notch 124, while rounded knob 125 is aligned with notch 164, as shown in FIG. 4, thereby securing first retainer 120 to second retainer 160. In this embodiment, the first and second retainers are matingly held together because inner sheath 40 and/or sliding ring 70 at least partially overlaps with both retainers, thereby inhibiting movement of the retainers with respect to each other.

In operation, clip device 110 is advanced to a target site through a working channel of an endoscope (not shown). The clip device is advanced in the state depicted in FIG. 4, with the exception that outer sheath 30 is distally advanced to cover arms 16 of clip 12 to constrain the clip within the delivery device. When the desired positioning is established, outer sheath 30 is retracted proximally to expose clip 12 and permit radial expansion of arms 16, as depicted in FIG. 4. In a next step, inner sheath 40 is advanced distally to abut sliding ring 70, causing the sliding ring to be advanced distally towards clip 12 and causing the arms of clip 12 to close radially inward to grasp tissue and promote hemostasis.

In a next step, inner sheath 40 is retracted proximally past first retainer 120 and second retainer 160, thereby exposing the coupling region between the retainers. At this time, since the retainers are no longer radially constrained, they will releasably detach from one another. It is important to note that since the engaging portions of the retainers are rounded knobs, it may be less likely that the retainers will get caught on one another after deployment. First retainer 120, which is attached to clip 12, remains inside the body. Second retainer 160, which is attached to operating wire 50, is retracted via the operating wire.

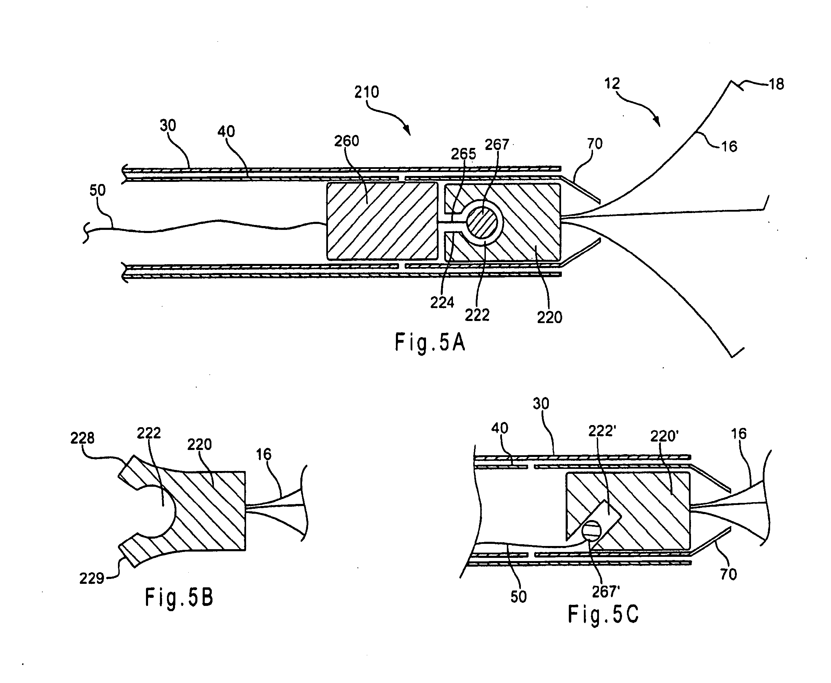

Referring now to FIGS. 5A-5C, further alternative embodiments for releasably securing and deploying clip 12 are provided. Clip device 210 comprises first retainer 220 and second retainer 260. First retainer 220 is operably attached to arms 16 of clip 12, while second retainer 260 is attached to the distal end of operating wire 50, as generally described above. Further, first retainer 220 has socket 222 formed therein, which preferably comprises a hole formed laterally therethrough. Channel 224 is disposed between socket 222 and the proximal end of first retainer 220, as shown in FIG. 5A.

First retainer 220 further comprises proximal arms 228 and 229, through which channel 224 extends. In a preferred embodiment, proximal arms 228 and 229 have a relaxed or biased state in which they are bowed radially outward, as shown in FIG. 5B. In this state, channel 224 is significantly opened.

Second retainer 260 has wire 265 coupled to its distal end, and further comprises ball 267 attached to wire 265, as shown in FIG. 5A. During delivery of the device, wire 265 fits within channel 224, while ball 267 fits within socket 222, as depicted in FIG. 5A. Therefore, first retainer 220 is coupled to second retainer 260. The first and second retainers are securely held together because inner sheath 40 and/or sliding ring 70 at least partially overlaps with both retainers, thereby inhibiting outward movement of the retainers, and in particular, proximal arms 228 and 229 of first retainer 220.

Clip device 210 is advanced to a target site through a working channel of an endoscope, as generally described above. During deployment, outer sheath 30 is retracted proximally to expose clip 12 and permit radial expansion of arms 16, as shown in FIG. 5A. Inner sheath 40 then is advanced distally to abut sliding ring 70, causing the sliding ring to be advanced distally towards clip 12 arid causing the arms of clip 12 to close inward to grasp tissue and promote hemostasis, as described above. In a next step, inner sheath 40 is retracted proximally past first retainer 220 and second retainer 260, thereby exposing the coupling region between the retainers. At this time, since proximal arms 228 and 229 are no longer radially constrained, they assume the configuration shown in FIG. 5B and permit ball 267 to detach from socket 222. First retainer 220, which is attached to clip 12, remains inside the body, while second retainer 260 is retracted via operating wire 50. In an alternative embodiment, second retainer 260 is eliminated and ball 267 is connected directly to operating wire 50.

In a further alternative embodiment, and as illustrated in FIG. 5C, first retainer 220' comprises angled channel 222' formed therein. Angled channel 222' may be formed partially through first retainer 220', or bored all the way through. Preferably, angled channel 222' is formed partially through the proximal end of first retainer 222', thereby forming a space through which operating wire 50 may extend. The distal end of operating wire 50 is coupled to ball 267', which is captured within channel 222' when covered by inner sheath 40 or sliding ring 70, as depicted in FIG. 5C. Once sliding ring 70 is advanced distally and/or inner sheath 40 retracted proximally, operating wire 50 may be retracted proximally and ball 267' will exit the proximal end of angled channel 222' to disengage the clip from the delivery apparatus.

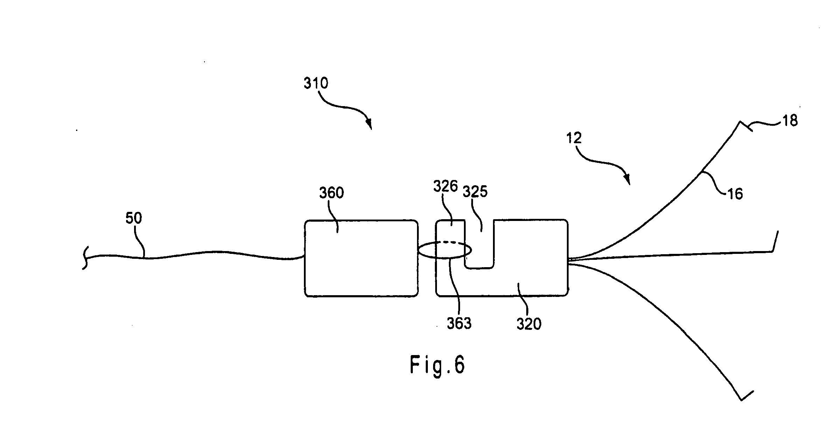

Referring now to FIG. 6, a side view of a further alternative mechanism for deploying clip 12 is provided. Clip device 310 comprises first retainer 320 and second retainer 360, which are releasably secured together by loop member 363. For illustrative purposes, outer sheath 30, inner sheath 40, and sliding ring 70 are omitted from FIG. 6, although they preferably are provided in accordance with the embodiments described above. In this embodiment, first retainer 320 preferably comprises notch 325 formed therein and has hook member 326 disposed proximal to the notch, as shown in FIG. 6. Second retainer 360 has a proximal end attached to operating wire 50, and has a distal end having loop member 363 extending therefrom.

In operation, loop member 363 is placed over hook member 326, as shown in FIG. 6, thereby securely coupling first retainer 320 to second retainer 360. Sliding ring 70 is advanced over at least notch 325 to ensure that loop member 363 cannot be inadvertently detached. Clip device 310 then is advanced to a target site through a working channel of an endoscope, as generally described above. During deployment, outer sheath 30 is retracted proximally to expose clip 12 and permit radial expansion of arms 16. Inner sheath 40 is advanced distally to abut sliding ring 70, causing the sliding ring to be advanced distally towards clip 12 and causing the arms of clip 12 to close inward, as described above. Inner sheath 40 then is retracted proximally to uncover first retainer 320 and second retainer 360. At this time, loop member 363 is no longer radially constrained about hook member 326, which permits first retainer 320 to disengage from second retainer 360. The proximal face of hook member 326 can be angled to facilitate movement of loop member 363 out of notch 325. After the retainers have separated, all of the components (except clip 12 attached to first retainer 320) are removed through the working channel of the endoscope.

In an alternative embodiment to FIG. 6, second retainer 360 may be eliminated and operating wire 50 may comprise a loop member, i.e., similar to loop member 363, at its distal end. In this case, the loop member of operating wire 50 is directly coupled to hook member 326 of first retainer 320.

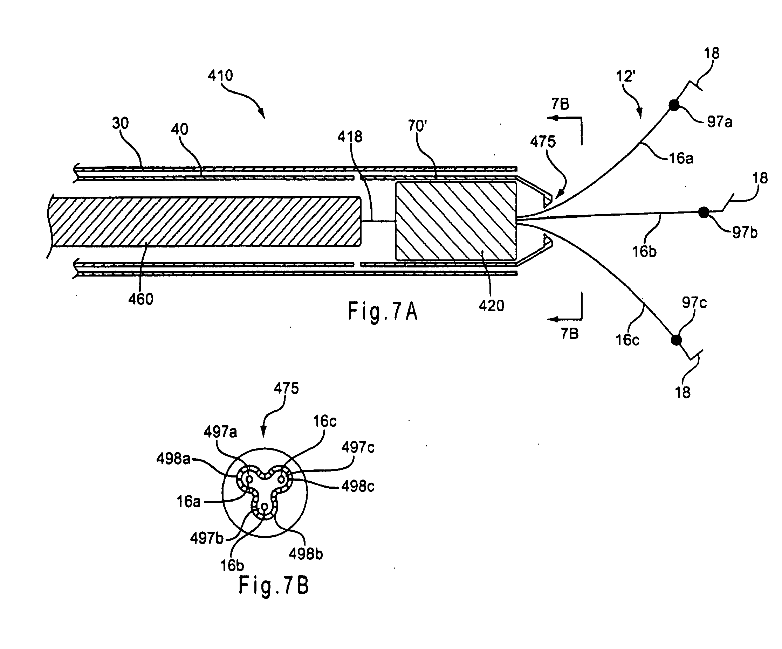

Referring now to FIGS. 7A-7B, a further alternative embodiment for releasably securing and deploying a clip device is provided. In FIG. 7A, clip device 410 comprises first retainer 420 and second retainer 460, which are releasably secured together by frangible element 418. The frangible element is designed to break apart in a controlled manner when a sufficient tensile force is imposed upon it, as explained in more detail below. In FIG. 7A, second retainer 460 is shown in the form of a cable that extends proximally within inner sheath 40. If desired, operating wire 50 may be coupled to a proximal region of second retainer 460 in a fashion similar to the other embodiments described above. Alternatively, second retainer 460 may be omitted and operating wire 50 may be coupled directly to first retainer 420, wherein operating wire 50 may comprise an integrally formed, frangible distal region.

Further, in this embodiment, clip 12' comprises three arms 16a, 16b and 16c having stop elements 97a. 97b and 97c, respectively. The stop elements preferably comprise a bead-shaped, oval-shaped, or circular-shaped metal material, or any other suitable shape. The stop elements may be disposed on an outer surface of one or more of arms 16a, 16b and 16c and soldered or otherwise attached proximal to ends 18 of the arms. Alternatively, the stop elements may be formed integrally with their respective arms during manufacture. Stop elements 97a, 97b and 97c serve multiple purposes. One purpose is to ensure that sliding ring 70' cannot be advanced over the distal end of clip 12'. Another purpose is to limit the amount of closing force that can be applied to arms 18 of clip 12'. Still another purpose of the stop elements is to engage distal end 475 of sliding ring 70' to facilitate disengagement of the first retainer from the second retainer, e.g., when retracting the second retainer with respect to the first retainer, or rotating the retainers with respect to each other, as explained more fully below.

When the stop elements are employed, distal end 475 of sliding ring 70' preferably comprises three channels 497a, 497b and 497c (see FIG. 7B) which are configured to permit movement of arms 16a, 16b and 16c therethrough, respectively. However, stop elements 97a, 97b and 97c are sized so that they cannot pass completely through the channels. Therefore, when sliding ring 70' is advanced distally over clip 12', arms 16a, 16b and 16c pass through channels 497a, 497b and 497c, respectively, but the stop elements serve as distal stop elements to ensure that the sliding ring cannot pass distally over the clip.

Sliding ring 70' comprises depressions 498a, 498b and 498c, which extend from the distal tip of sliding ring 70' into channels 497a, 497b and 497c, respectively (see FIG. 7B). Stop elements 97a, 97b and 97c preferably are sized to be at least partially seated within depressions 498a, 498b and 498c, respectively. In one embodiment, the stop elements may lockingly engage their respective depressions, e.g., using a snap-fit, thereby ensuring that sliding ring 70' cannot disengage from clip 12'.

In operation, clip device 410 is advanced to a target site through a working channel of an endoscope, as generally described above. The proximal end of first retainer 420 is coupled to the distal end of second retainer 460 using frangible element 418. During deployment, outer sheath 30 is retracted proximally to expose clip 12 and permit radial expansion of arms 16. Inner sheath 40 is advanced distally to abut sliding ring 70', causing the sliding ring to be advanced distally towards clip 12' and causing the arms of clip 12' to close inward, as described above. Stop elements 97a, 97b and 97c engage depressions 498a, 498b and 498c, respectively, to ensure that the sliding ring is not advanced distally past the end of the clip.

In a next step, inner sheath 40 is held steady while second retainer 460 (or operating wire 50 coupled to second retainer 460) is retracted proximally. The retraction of second retainer 460 with respect to first retainer 420 imposes a tensile force upon frangible element 418, thereby breaking the frangible element and detaching the retainers. Based on tactile feedback, a physician will be able to sense when the frangible element has been broken and the retainers have detached.

It should be noted that during proximal retraction of second retainer 460, clip 12' will be held steady and not displaced from engagement with the tissue. Specifically, after sliding ring 70' has been advanced distally and has engaged stop elements 97a, 97b and 97c, the stop elements prohibit proximal retraction of clip 12' with respect to sliding ring 70'. Since inner sheath 40 is held steady and prevents proximal retraction of sliding ring 70', clip 12' cannot be retracted proximally, either. This helps prevent excessive forces from being applied to the tissue.

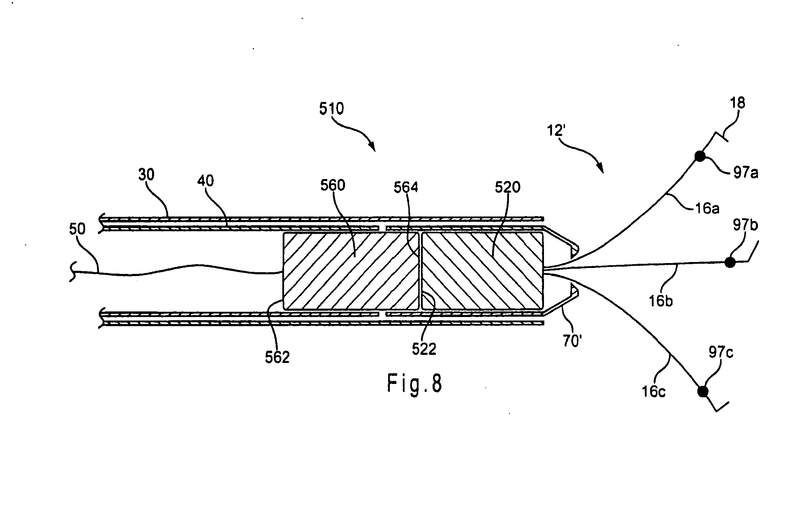

Referring now to FIG. 8, a further alternative embodiment for releasably securing and deploying a clip, such as clip 12', is provided. In FIG. 8, clip 12' and sliding ring 70' preferably are provided as described in FIGS. 7A-7B above. Therefore, clip 12' comprises stop elements 97a, 97b and 97c, which are sized to be at least partially be seated within depressions 498a, 498b and 498c, respectively, at the distal end of sliding ring 70' (see FIG. 7B).

Clip device 510 comprises first retainer 520 and second retainer 560, which are releasably secured together by magnetic forces, i.e., first retainer 520 has a first magnetic force and second retainer 560 has an opposing magnetic force. In operation, inner sheath 40 is advanced distally to cause sliding ring 70' to close arms 16a, 16b and 16c. When sliding ring 70' is advanced distally, stop elements 97a, 97b and 97c of clip 12' engage the depressions in sliding ring 70'. Inner sheath 40 then is held steady while operating wire 50 is retracted proximally, thereby overcoming the magnetic force and causing second retainer 560 to detach from first retainer 520. In effect, distal end 564 of second retainer 560 separates from proximal end 522 of first retainer 520, and second retainer 560 becomes retracted further proximally within inner sheath 40. After the retainers have separated, inner sheath 40 is retracted proximally, and all of the components (except clip 12' attached to First retainer 520) are removed through the working channel of the endoscope.

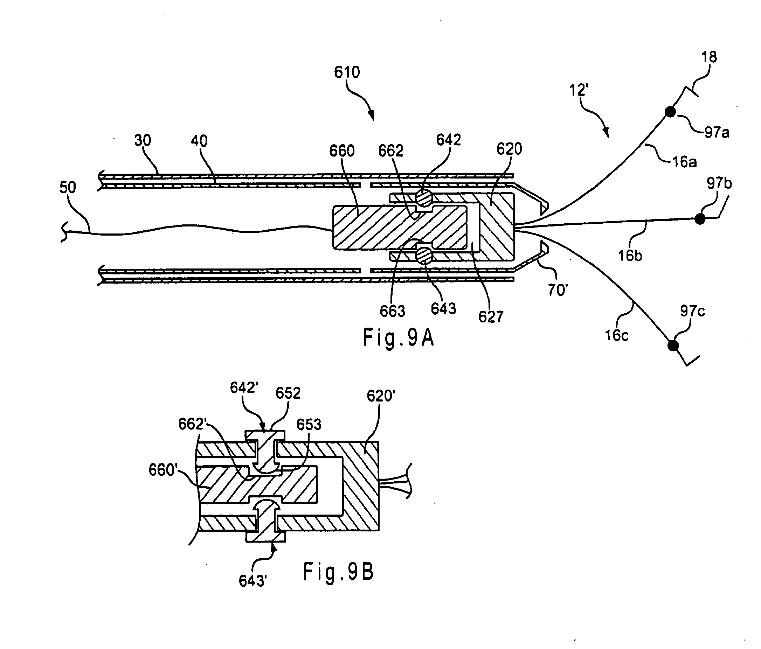

Referring now to FIGS. 9A-9B, further alternative embodiments for deploying a clip, such as clip 12', are provided. In FIG. 9A, clip 12' and sliding ring 70' preferably are provided as described in FIGS. 7A-7B above. Therefore, clip 12' comprises stop elements 97a, 97b and 97c, which are sized to be at least partially be seated within depressions 498a, 498b and 498c, respectively, at the distal end of sliding ring 70' (see FIG. 7B).

Clip device 610 comprises first retainer 620 and second retainer 660, which are releasably secured together by a ball bearing and detent arrangement. Specifically, first retainer 620 has inner bore 627 formed in its proximal end. Ball elements 642 and 643 are coupled to opposing exterior regions of first retainer 620 and partially extend into bore 627, as shown in FIG. 9A. The ball elements also extend radially outward towards sliding ring 70', and preferably contact the sliding ring, as depicted in FIG. 9A. Ball elements 642 and 643 are movable, but not removable, relative to first retainer 620.

Second retainer 660 has an outer diameter that is less than the diameter of bore 627 of first retainer 620, thereby allowing second retainer 660 to be disposed within the bore. Second retainer 660 also has opposing notches 662 and 663 formed therein, which are sized to receive an outer portion of ball elements 642 and 643, respectively, as described below.

In operation, clip device 610 is advanced to a target site through a working channel of an endoscope, as generally described above. During advancement, sliding ring 70' and/or inner sheath 40 are disposed over ball elements 642 and 643, thereby urging the ball elements in an inward direction into a portion of notches 662 and 663, respectively. When urged radially inward towards the notches, ball elements 642 and 643 substantially prohibit longitudinal movement of first retainer 620 with respect to second retainer 660, as shown in FIG. 9A.

During deployment, outer sheath 30 is retracted proximally to expose clip 12' and permit radial expansion of arms 16. Inner sheath 40 is advanced distally to abut sliding ring 70', causing the sliding ring to be advanced distally towards clip 12' and causing the arms of clip 12' to close inward, as described above. Inner sheath 40 then is retracted proximally past second retainer 660. When sliding ring 70' and/or inner sheath 40 no longer constrain ball elements 642 and 643, the ball elements are permitted to move radially outward, i.e., out of notches 662 and 663. At this time, second retainer 660 may be retracted proximally via operating wire 50, and ball elements 642 and 643 will not catch on their respective detents. Alternatively, ball elements 642 and 643 may be deformable when subjected to a sufficient tensile release force.

The embodiment of FIG. 9B is similar to that described in FIG. 9A, with a main exception that one or more rivet elements 642' and 643' are employed in lieu of ball elements 642 and 643. Rivet element 642' preferably comprises a first end having flat surface 652 and a second end having enlarged rounded region 653. A smaller diameter portion extends between flat surface 652 and rounded region 653. The smaller diameter portion is disposed through a hole in first retainer 620', as shown in FIG. 9B, to contain rivet element 642'. In operation, when sliding ring 70' and/or inner sheath 40 are disposed over first retainer 620', rivet element 642' is urged radially inward, thereby urging rounded region 653 into notch 662' in second retainer 660' to secure the first retainer to the second retainer. When sliding ring 70' and/or inner sheath 40 no longer constrain rivet 642', it may move radially outward and will not catch on notch 662'. Therefore, second retainer 660' may disengage from first retainer 620'.

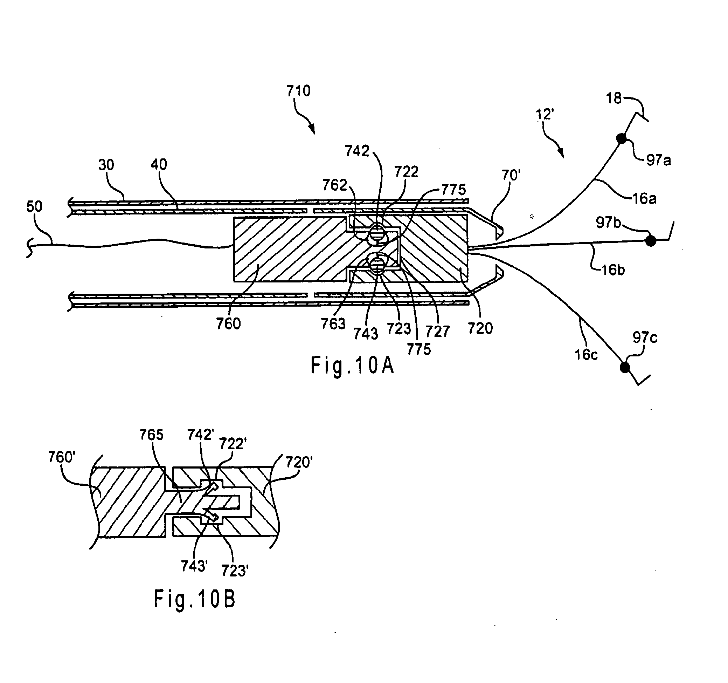

Referring now to FIGS. 10A-10B, variations on the embodiment described in FIGS. 9A-9B are shown. In FIG. 10A, clip device 710 preferably comprises two opposing ball elements 742 and 743 that selectively permit coupling of first retainer 720 and second retainer 760.

First retainer 720 has inner bore 727 formed in its proximal end, which is adapted to receive a reduced diameter distal region of second retainer 760, as shown in FIG. 10A. First retainer 720 further comprises first and second notches 722 and 723 formed in bore 727, while the distal region of second retainer 760 has recesses 762 and 763 formed therein. Recesses 762 and 763 are configured to contain a substantial portion of ball elements 742 and 743, respectively, while a portion of the ball elements may extend outside of the confines of the recesses, as depicted in FIG. 10A. The recesses are configured, however, to never permit the ball elements to escape therefrom.

In a preferred embodiment, biasing means 775, e.g., a compression spring, is disposed within recess 762. The biasing means is disposed beneath ball element 742 to bias the ball element radially outward, i.e., towards notch 722. A second biasing means (not shown) preferably is used to bias ball element 743 radially outward in the same manner.

In operation, clip device 710 is advanced to a target site through a working channel of an endoscope, as generally described above. During advancement, ball elements 742 and 743 are aligned with notches 722 and 723, respectively. The biasing means bias their respective ball elements radially outward into their respective notches to securely couple first retainer 720 to second retainer 760.

After deployment of clip 12', inner sheath 40 is advanced distally and held steady against sliding ring 70'. At this time, second retainer 760 may be retracted proximally via operating wire 50. Stop elements 97a, 97b and 97c may engage depressions 498a, 498b and 498c, respectively, in sliding ring 70' (see FIG. 7B). The intentional retraction of second retainer 760 by a physician will overcome the force provided by biasing means 775, thereby causing ball elements 742 and 743 to be forced radially inward and permitting disengagement of the two retainers. Alternatively, ball elements 742 and 743 may be deformable when subjected to a sufficient tensile release force. Once detached, second retainer 760 may be retracted via inner sheath 40, while first retainer 720 attached to clip 12' is left inside the patient.

The embodiment of FIG. 10B is similar to that described in FIG. 10A, with a main exception that one or more biased elements 742' and 743' are employed in lieu of ball elements 742 and 743. Biased elements 742' and 743' preferably are integrally formed with reduced diameter distal region 765 of second retainer 760', as shown in FIG. 10B. Biased elements 742' and 743' have a predetermined configuration in which they are biased radially outward into notches 722' and 723', respectively, to secure second retainer 760' to first retainer 720'. When it is desired to disengage the retainers, second retainer 760' is retracted proximally with respect to first retainer 720' to urge biased elements 742' and 743' radially inward, i.e., out of notches 722' and 723'. Therefore, second retainer 760' may disengage from first retainer 720'.

In the embodiments of FIGS. 9-10, it will be apparent that although two opposing ball elements are shown, only one ball element may be employed, or alternatively, three or more may be used. Additionally, while ball-shaped elements are depicted, it will be apparent that these elements may comprise other shapes, such as oval-shaped elements, cone-shaped elements, and so forth.

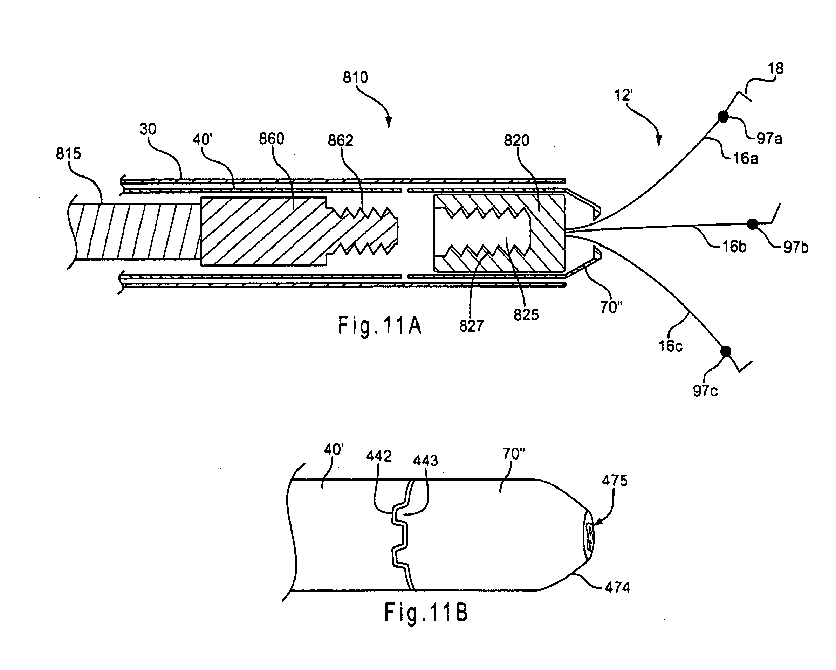

Referring now to FIGS. 11A-11B, a further alternative embodiment of the present invention is described. In FIGS. 11A-11B, clip 12' comprises stop elements 97a, 97b and 97c, which are sized to be at least partially be seated within depressions 498a, 498b and 498c, respectively, at distal end 475 of sliding ring 70' (see FIG. 7B). Clip device 810 comprises first retainer 820 and second retainer 860. First retainer 820 has bore 825 formed in its proximal end. Bore 825 has internal threading 827, which is configured to releasably mate with external threading 862, which is disposed on a distal region of second retainer 860. Torque cable 815 is coupled to a proximal region of second retainer 860 and preferably spans the entire length of the delivery system.

In the embodiment of FIGS. 11A-11B, inner sheath 40' and sliding ring 70" are similar to the embodiments described above. However, the distal end of inner sheath 40' is configured to mate with the proximal end of sliding ring 70" to inhibit rotational movement therebetween, for purposes explained below. In one embodiment, the distal end of inner sheath 40' comprises at least one notch 442 that is configured to mate with at least one corresponding knob 443 extending from the proximal end of sliding ring 70", as shown in FIG. 11B.

In operation, clip device 810 is advanced to a target site through a working channel of an endoscope, as generally described above. During advancement, first retainer 820 is secured to second retainer 860 by engaging their respective internal and external threaded regions. After clip 12' is deployed, as described above, torque cable 815 is rotated in a direction that causes the threaded regions to disengage. Once the first and second retainers are disengaged, torque cable 815 and second retainer 860 may be retracted proximally via inner sheath 40', while first retainer 820 attached to clip 12' is left inside the patient.

In this particular embodiment, once sliding ring 70" has been advanced distally by inner sheath 40', stop elements 97a, 97b and 97c engage the depressions in sliding ring 70". As noted above, the stop elements may lock into engagement with the depressions in sliding ring 70", e.g., using a snap-fit. Further, notches 442 of inner sheath 40' mate with corresponding knobs 443 of sliding ring 70" (see FIG. 11B) to prevent rotational movement of the inner sheath with respect to the sliding ring. Therefore, by holding inner sheath 40' steady while rotating torque cable 815 relative thereto, second retainer 860 is rotated with respect to first retainer 820, thereby causing the retainers to disengage. In other words, by holding inner sheath 40' rotationally steady, sliding ring 70" cannot rotate (see FIG. 11B), and therefore, clip 12' cannot rotate because stop elements 97a, 97b and 97c are restrained within the depressions 498a, 498b and 498c of the rotationally-steady sliding ring.

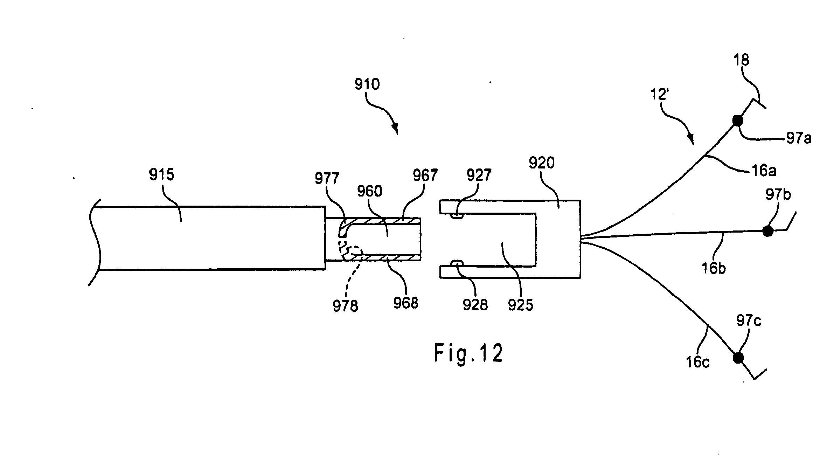

Referring now to FIG. 12, a further alternative embodiment of the present invention is described. In FIG. 12, clip device 910 comprises first retainer 920 and second retainer 960. For illustrative purposes, the outer sheath, inner sheath, and sliding ring are omitted from FIG. 12. First retainer 920 has bore 925 formed in its proximal end, and further comprises first and second inwardly-directed knobs 927 and 928 projecting into bore 925, as shown in FIG. 12. Second retainer 960 has proximal and distal ends, and further has an outer diameter that is slightly smaller than an inner diameter of bore 925. Axial channels 967 and 968 are formed in the distal end of second retainer 960, preferably 180 degrees apart, and extend longitudinally from the distal end towards the proximal end, as shown in FIG. 12. Before reaching the proximal end, axial channel 967 transitions into circumferential channel 977, which preferably extends about 90 degrees around the outer circumference of second retainer 960. Similarly, axial channel 968 transitions into circumferential channel 978, which extends about 90 degrees around the outer circumference of second retainer 960, as shown in FIG. 12.

Channels 967, 968, 977 and 978 preferably are etched into an exterior surface of second retainer 960, which may be formed of stainless steel or the like. Knob 927 of first retainer 920 is sized for movement within channels 967 and 977, while knob 928 is sized for movement within channels 968 and 978, as described below.

In operation, clip device 910 is advanced to a target site through a working channel of an endoscope, as generally described above. During advancement, first retainer 920 is secured to second retainer 960 by aligning knobs 927 and 928 with axial channels 967 and 968, respectively. Second retainer 960 is moved towards first retainer 920 to cause the knobs to slide within their respective axial channels. When knobs 927 and 928 reach the proximal portion of their respective axial channels, second retainer 960 is rotated about 90 degrees with respect to first retainer 920, thereby causing knobs 927 and 928 to be advanced within their respective circumferential channels 977 and 978. In this state, first and second retainers 920 and 960 are coupled together, and longitudinal movement of the retainers with respect to each other is substantially prohibited.

Clip 12' may then be deployed and secured to tissue by advancing sliding ring 70" of FIG. 11B. In a next step, torque cable 915, which is operably coupled to the proximal end of second retainer 960, is rotated about 90 degree in a direction opposite the direction used to lock the retainers together. This rotation causes knobs 927 and 928 to be aligned with axial channels 967 and 968, respectively. At this time, second retainer 960 may be retracted proximally to cause knobs 927 and 928 to slide within axial channels 967 and 968, respectively, thereby unlocking the retainers. Once the first and second retainers are disengaged, torque cable 915 and second retainer 960 may be retracted proximally through inner sheath 40, while first retainer 920 attached to clip 12 is left inside the patient. It will be apparent that although two opposing knobs are shown in FIG. 12, only one knob/channel arrangement may be employed, or alternatively, three or more may be used.

In this embodiment, clip device 910 preferably employs clip 12', inner sheath 40' and sliding ring 70", as described in FIGS. 11A-11B above. As noted above, the use of such interlocking components will hold clip 12' rotationally stationary while second retainer 960 is rotated with respect to first retainer 920.

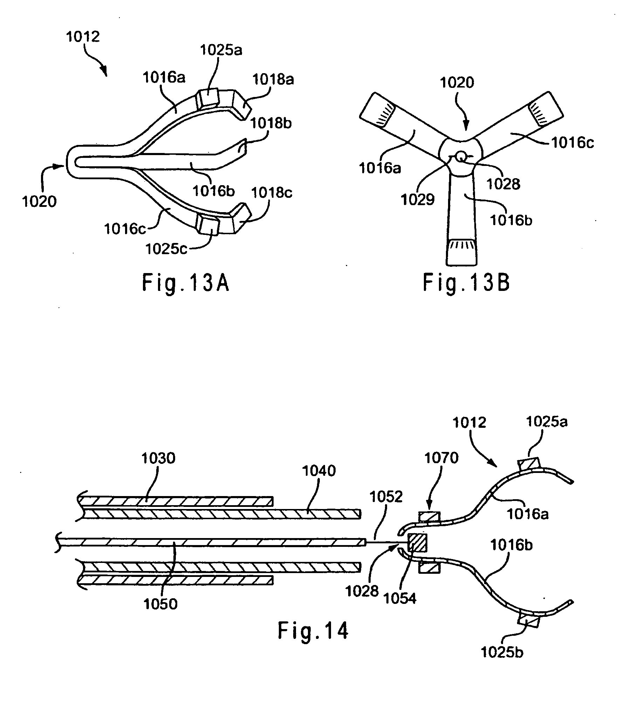

Referring now to FIGS. 13-17, various alternative embodiments of the present invention are described. Alternative clip 1012 comprises at least two arms, and in the embodiment of FIGS. 13A-13B, comprises three arms 1016a, 1016b and 1016c, each having proximal and distal ends. The distal ends of arms 1016a, 1016b and 1016c comprise bends 1018a, 1018b and 1018c, respectively, which are configured to engage tissue.

In general, clip 1012 is similar to clip 12, described above, with the main exception that arms 1016a, 1016b and 1016c comprise substantially flat regions along part or all of their length, as shown in FIGS. 13-13B. Moreover, the proximal ends of arms 1016a, 1016b and 1016c unite to form proximal end 1020 of clip 1012. Clip 1012 may be formed by cutting a flat clip having the desired number of arms (e.g., three) from a planar sheet of material, then bending the arms into the desired final shape. Proximal end 1020 has hole 1028 disposed therein, as shown in FIG. 13B. Optionally, at least one slit 1029 may be formed around the circumference of hole 1028, for purposes described below.

Referring now to FIG. 14, a first method of using clip 1012 of FIGS. 13A-13B is described. The apparatus comprises outer sheath 1030 and inner sheath 1040, which are similar to outer sheath 30 and inner sheath 40, as described above. The distal end of inner sheath 1040 is configured to engage collet 1070, which is disposed about the proximal end of clip 1012 and designed to close the clip, as explained below. Preferably, arms 1016a, 1016b and 1016c comprise distal stop members 1025a, 1025b and 1025c, as shown in FIG. 13A and FIG. 14. The distal stop members ensure that collet 1070 cannot be advanced distally over the clip. Collet 1070 is similar in design and function to sliding ring 70, 70' of the above-described embodiments.

In FIG. 14, clip 1012 is coupled to operating wire 1050 prior to deployment. The distal end of operating wire 1050 is coupled to frangible member 1052, which in turn is coupled to knob 1054, as shown in FIG. 14. Alternatively, frangible member 1052 may be integrally formed at the distal end of operating wire 1050. Frangible member 1052 extends through hole 1028 in proximal end 1020 of clip 1012, such that knob 1054 is confined distal to hole 1028, as shown in FIG. 14.

Clip 1012 is advanced to a target site with arms 1016a, 1016b and 1016c radially restrained by outer sheath 1030. Outer sheath 1030 is retracted to cause arms 1016a, 1016b and 1016c to deploy radially outward, as shown in FIG. 14 and generally described above. In a next step, inner sheath 1040 is advanced distally to abut collet 1070 and distally advance collet 1070 over arms 1016a, 1016b and 1016c. The arms are urged radially inward to engage tissue and promote hemostasis.

When collet 1070 abuts distal stop members 1025a, 1025b and 1025c, inner sheath 1040 is held steady while operating wire 1050 is retracted proximally. At this time, knob 1054 engages hole 1028 but cannot be pulled through the hole. The tensile force causes frangible member 1052 to break, thereby separating clip 1012 from operating wire 1050. The outer sheath, inner sheath and operating wire are then removed from the patient.

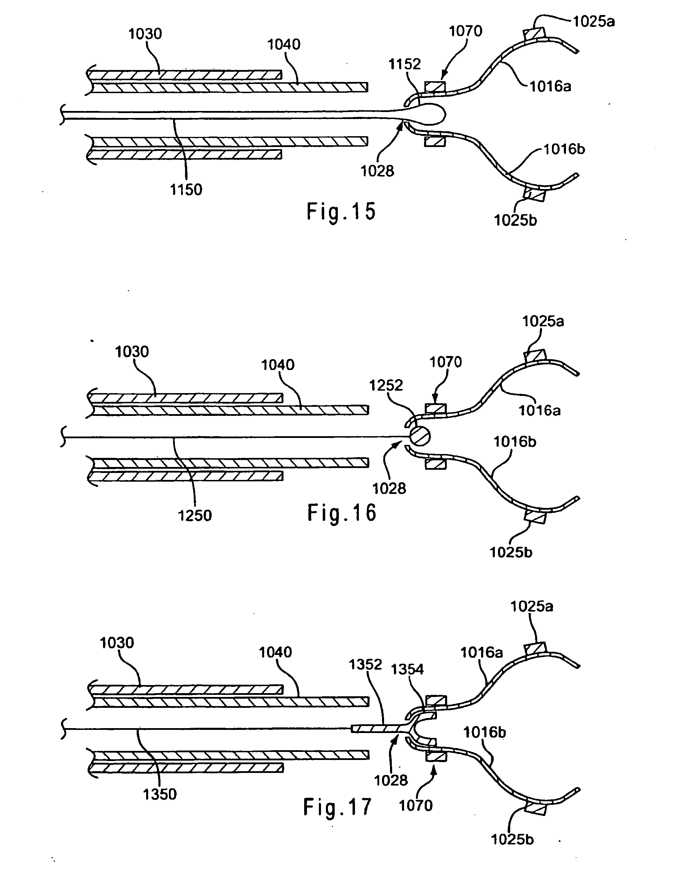

Referring now to FIGS. 15-17, various alternative release mechanisms for deploying clip 1012 of FIGS. 13A-13B are described. In FIG. 15, wire 1150 is advanced distally through hole 1028 of clip 1012, then loop 1152 is formed, and wire 1150 is pulled back through hole 1028. In a relaxed state, loop 1152 is secured distal to hole 1028, i.e., the loop will not pull through the hole in the absence of a significant force. In use, collet 1070 is advanced via inner sheath 1040 and abuts distal stop members 1025a, 1025b and 1025c. Inner sheath 1040 then is held steady while wire 1150 is retracted proximally. At this time, an inwardly directed force causes loop 1152 to compress and pull through hole 1028, thereby separating clip 1012 from wire 1150.

In FIG. 16, the distal end of operating wire 1250 extends through hole 1028 and is coupled to knob 1252, which is disposed distal to hole 1028. In use, collet 1070 is advanced via inner sheath 1040 and abuts distal stop members 1025a, 1025b and 1025c. Inner sheath 1040 then is held steady while wire 1250 is retracted proximally. At this time, knob 1252 pulls through hole 1028, thereby separating clip 1012 from operating wire 1050. Preferably, in this embodiment, at least one slit 1029 (see FIG. 13B) is employed to facilitate retraction of knob 1252 through hole 1028.

In FIG. 17, the distal end of operating wire 1350 is coupled to deformable member 1354. In this example, deformable member 1354 comprises at least two arms that extend radially outward in a relaxed state. The arms of deformable member 1354 may be coupled to rigid proximal section 1352, which in turn is coupled to operating wire 1350, as shown in FIG. 17. Alternatively, operating wire 1350 may be coupled directly to deformable member 1354.

In use, proximal section 1352 (or operating wire 1350) or is disposed through hole 1028, while deformable member 1354 is disposed distal to hole 1028, as shown in FIG. 17. Collet 1070 is advanced via inner sheath 1040 and abuts distal stop members 1025a, 1025b and 1025c, and inner sheath 1040 then is held steady while operating wire 1350 is retracted proximally. At this time, the arms of deformable member 1354 are urged radially inward to pull deformable member 1354 through hole 1028, thereby separating clip 1012 from operating wire 1350. In this embodiment, at least one slit 1029 (see FIG. 13B) may be employed to facilitate retraction of deformable member 1354 through hole 1028.

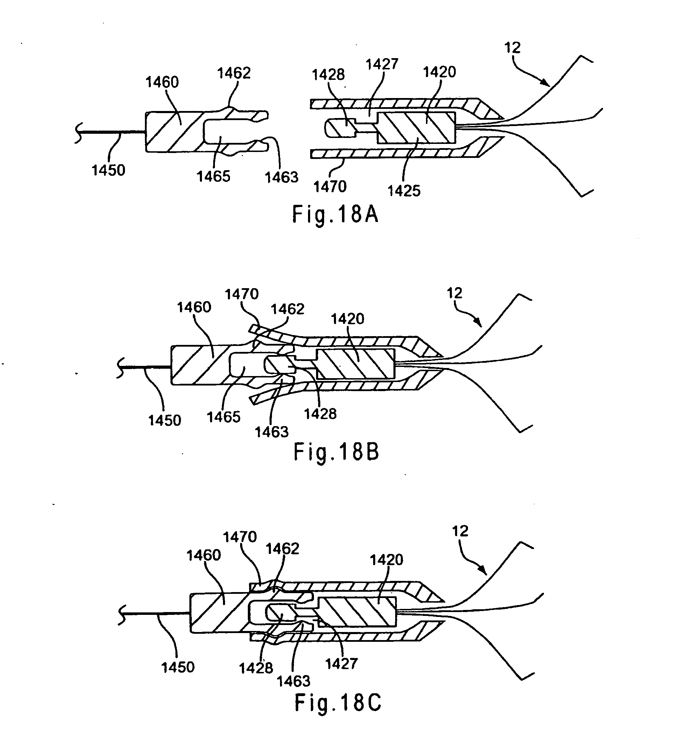

Referring now to FIGS. 18A-18C, a further alternative clip retainer system is shown. First retainer 1420 has proximal and distal regions 1428 and 1425. Distal region 1425 comprises a generally cylindrical shape and is attached to clip 12. Proximal region 1428 preferably has a smaller diameter than distal region 1425, and may comprise a rounded proximal edge, as depicted in FIG. 18A. At least one notch 1427 is disposed between the proximal and distal regions, as shown in FIG. 18A.

Second retainer 1460 comprises a generally cylindrical body having proximal and distal regions. The proximal region is attached to operating wire 1450. The distal region comprises bore 1465 having at least one knob 1463 extending therein, as shown in FIG. 18A. Further, an exterior surface of second retainer 1460 has at least one protruding member 1462 extending radially outward, as depicted in FIG. 18A.

In the embodiment of FIGS. 18A-18C, sliding ring 1470 is disposed over first retainer 1420, as shown in FIG. 18A. Sliding ring 1470 comprises a flexible proximal region, as will be explained in FIG. 18B. Optionally, sliding ring 1470 may comprise a lateral slit (not shown) disposed on a proximal region to enhance its radial flexibility and accommodate second retainer 1460, as explained below.

In operation, a physician may attach second retainer 1460 to first retainer 1420 by distally advancing second retainer 1460. As shown in FIG. 18B, protruding member 1462 causes radial expansion of a proximal region of sliding ring 1470. At this time, knob 1463 is passed over proximal region 1428 of first retainer 1420, preferably with little or no resistance. As second retainer 1460 is further advanced, proximal region 1428 of first retainer 1420 is disposed within the confines of bore 1465. Moreover, this placement allows sliding ring 1470 to exert a resilient inward force upon protruding member 1462, thereby urging knob 1463 into notch 1427, as shown in FIG. 18C.

In a next step, an inner and outer sheath may be disposed over the apparatus and inserted into the patient, as generally set forth above. After the inner sheath advances sliding ring 1470 in a distal direction to close the arms of clip 12, second retainer 1460 may be retracted proximally via operating wire 1450 to allow knob 1463 to disengage from notch 1427, thereby separating the retainers.

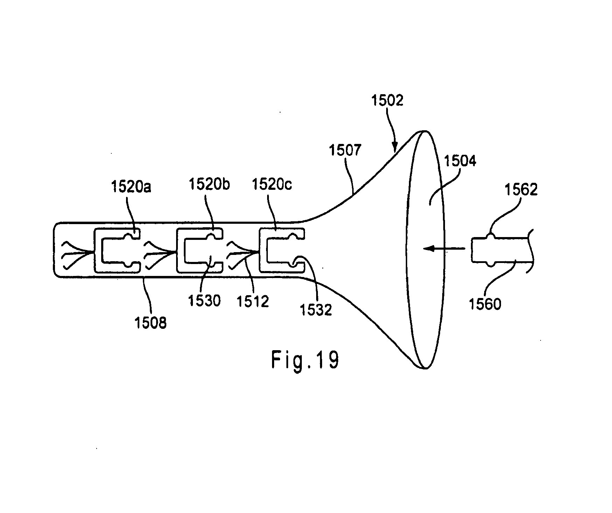

Referring now to FIG. 19, an apparatus that may be used to hold multiple clips is disclosed. Clip holder 1502 comprises proximal region 1508 and enlarged diameter distal region 1504. A taper 1507 is provided between the proximal and distal regions. Multiple clips 1520a, 1520b and 1520c are adapted to be pre-loaded into proximal region 1508, as shown in FIG. 19. The arms 1512 of clips 1520a-c may be nested within bores 1530 of adjacent clips, or disposed proximal to adjacent clips as shown. First retainers 1520a-c may comprise portions adapted to mate with complementary portions on second retainer 1560. For example, ball element 1562 may be adapted to engage with notch elements 1532 of second retainers 1520a-c. Advantageously, each time a new clip is needed, a physician may simply insert second retainer 1560 into clip holder 1502, engage a clip, and proceed to deploy the clip within a patient according the steps generally set forth above.

While various embodiments of the invention have been described, it will be apparent to those of ordinary skill in the art that many more embodiments and implementations are possible within the scope of the invention. Accordingly, the invention is not to be restricted except in light of the attached claims and their equivalents.

## Claims

1. A clip device (10) for use in endoscopic medical procedures comprising:
a clip (12) having a plurality of arms (16) each having a proximal end (14) and a distal end (18), wherein the proximal end of each of the arms is joined to a first retainer (20) and extends distally therefrom, each of the arms being formed of a resilient material and shaped so that the distal ends tend to be spaced apart from each other when the clip is in an open position and adjacent to each other when the clip is in a closed position;
a sliding ring (70) being movable between a first position when the clip is in the open position and a second position to hold the arms of the clip in the closed position; and
at least one stop element (97a-97c) disposed on at least one of the arms of the
clip,
wherein the sliding ring has a distal end having at least one channel (497a-497c) formed therein, wherein the channel is sized to permit movement of one of the plurality of arms therethrough and inhibit movement of the stop element therethrough; **characterized by**
at least one depression (498a-498c) formed in the distal end of the sliding ring and configured to engage the stop element disposed on the arm.

2. The clip device of claim 1, wherein the stop element limits distal movement of the sliding ring.

3. The clip of claim 1 wherein the stop element is sized to be at least partially seated within the depression.

4. The clip device of claim 1, wherein the stop element comprises at least one bead-shaped element disposed on the arm.

5. The clip device of claim 1 wherein the at least one depression lockingly engages the stop element disposed on the arm.

6. The clip device of claim 1 wherein the first retainer has proximal and distal regions, the clip device further comprising a second retainer having proximal and distal regions, wherein the first retainer is configured to be coupled to the second retainer prior to deployment of the clip, and wherein the first retainer is configured to be disengaged from the second retainer after the clip is deployed.

7. The clip device of claim 6 wherein the first retainer is configured to be disengaged from the second retainer by proximally retracting the second retainer with respect to the first retainer.

8. The clip device of claim 7 wherein the first retainer and the second retainer have opposing magnetic forces, wherein application of a sufficient retraction force to the second retainer causes the second retainer to disengage from the first retainer.

9. The clip device of claim 7 wherein the first retainer is coupled to the second retainer using a ball bearing arrangement.

10. The clip device of claim 6 further comprising:
a wire extending from the distal region of the second retainer and a ball attached to a distal end of the wire; and
a channel and socket formed in the proximal region of the first retainer, wherein the channel and socket are configured to receive the wire and ball, respectively, to couple the first retainer to the second retainer.

11. The clip device of claim 10 wherein the sliding ring prevents disengagement of the ball from the socket prior to deployment of the clip.

12. The clip device of claim 6 wherein the first retainer is disengaged from the second retainer by causing rotation of the second retainer with respect to the first retainer.

## Patentansprüche

1. Klammervorrichtung (10) zur Verwendung bei medizinischen Endoskopieverfahren mit
einer Klammer (12) mit mehreren Armen (16), die jeweils ein proximales Ende (14) und ein distales Ende (18) haben, wobei das proximale Ende jedes der Arme mit einem ersten Halter (20) verbunden ist und sich distal davon erstreckt, wobei jeder der Arme aus einem federnden Material gebildet und so gestaltet ist, dass die distalen Enden dazu neigen, voneinander beabstandet zu sein, wenn die Klammer in einer offenen Position ist, und einander benachbart zu sein, wenn die Klammer in einer geschlossenen Position ist,
einem Gleitring (70), der zwischen einer ersten Position, wenn die Klammer in der offenen Position ist, und einer zweiten Position bewegbar ist, um die Arme der Klammer in der geschlossenen Position zu halten, und
mindestens einem Anschlagelement (97a - 97c), das an mindestens einem der Arme der Klammer angeordnet ist,
wobei der Gleitring ein distales Ende mit mindestens einem darin gebildeten Kanal (497a - 497c) hat, wobei der Kanal so bemessen ist, um eine Bewegung eines der mehreren Arme dort hindurch zu gestatten und eine Bewegung des Anschlagelements dort hindurch zu verhindern, **dadurch gekennzeichnet, dass**
mindestens eine Einsenkung (498a - 498c) im distalen Ende des Gleitrings ausgebildet und so konfiguriert ist, dass sie das am Arm angeordnete Anschlagelement in Eingriff nimmt.

2. Klammervorrichtung nach Anspruch 1, wobei das Anschlagelement die distale Bewegung des Gleitrings begrenzt.

3. Klammervorrichtung nach Anspruch 1, wobei das Anschlagelement so bemessen ist, dass es mindestens teilweise in der Einsenkung sitzt.

4. Klammervorrichtung nach Anspruch 1, wobei das Anschlagelement mindestens ein am Arm angeordnetes perlenförmiges Element umfasst.

5. Klammervorrichtung nach Anspruch 1, wobei die mindestens eine Einsenkung das am Arm angeordnete Anschlagelement verriegelnd in Eingriff nimmt.

6. Klammervorrichtung nach Anspruch 1, wobei der erste Halter proximale und distale Bereiche hat, wobei die Klammervorrichtung ferner einen zweiten Halter mit proximalen und distalen Bereichen umfasst, wobei der erste Halter so konfiguriert ist, dass er vor dem Ausbringen der Klammer an den zweiten Halter gekoppelt ist, und wobei der erste Halter so konfiguriert ist, dass er nach dem Ausbringen der Klammer aus dem zweiten Halter ausgerückt ist.

7. Klammervorrichtung nach Anspruch 6, wobei der erste Halter so konfiguriert ist, dass er aus dem zweiten Halter ausgerückt wird, indem der zweite Halter bezüglich des ersten Halters proximal zurückgezogen wird.

8. Klammervorrichtung nach Anspruch 7, wobei der erste Halter und der zweite Halter entgegengesetzte Magnetkräfte haben, wobei durch die Ausübung einer ausreichenden Rückzugkraft auf den zweiten Halter veranlasst wird, dass der zweite Halter aus dem ersten Halter ausrückt.

9. Klammervorrichtung nach Anspruch 7, wobei der erste Halter unter Verwendung einer Kugellageranordnung an den zweiten Halter gekoppelt ist.

10. Klammervorrichtung nach Anspruch 6, ferner mit einem Draht, der sich vom distalen Bereich des zweiten Halters erstreckt, und einer Kugel, die an einem distalen Ende des Drahts angebracht ist, und einem Kanal und einer Pfanne, die im proximalen Bereich des ersten Halters ausgebildet und so konfiguriert sind, dass sie jeweils den Draht und die Kugel aufnehmen, um den ersten Halter an den zweiten Halter zu koppeln.

11. Klammervorrichtung nach Anspruch 10, wobei der Gleitring ein Ausrücken der Kugel aus der Pfanne vor dem Ausbringen der Klammer verhindert.

12. Klammervorrichtung nach Anspruch 6, wobei der erste Halter aus dem zweiten Halter ausgerückt wird, indem veranlasst wird, dass sich der zweite Halter bezüglich des ersten Halters dreht.

## Revendications

1. Dispositif d'agrafe (10) à utiliser dans des procédures médicales endoscopiques, comprenant:
une agrafe (12) comprenant une pluralité de bras (16) présentant chacun une extrémité proximale (14) et une extrémité distale (18), dans lequel l'extrémité proximale de chacun des bras est jointe à un premier élément de retenue (20) et s'étend de façon distale à partir de celui-ci, chacun des bras étant constitué d'un matériau élastique et configuré de telle sorte que les extrémités distales aient tendance à être espacées l'une de l'autre lorsque l'agrafe se trouve dans une position ouverte, et à proximité l'une de l'autre lorsque l'agrafe se trouve dans une position fermée;
un anneau coulissant (70) mobile entre une première position lorsque l'agrafe se trouve dans la position ouverte, et une deuxième position pour maintenir les bras de l'agrafe dans la position fermée; et
au moins un élément d'arrêt (97a-97c) disposé sur au moins un des bras de l'agrafe,
dans lequel l'anneau coulissant présente une extrémité distale comprenant au moins un canal (497a-497c) formé dans celle-ci, dans lequel le canal est dimensionné de manière à permettre le déplacement de l'un parmi la pluralité de bras à travers celui-ci et à empêcher le déplacement de l'élément d'arrêt à travers celui-ci,
**caractérisé par** au moins une dépression (498a-498c) formée dans l'extrémité distale de l'anneau coulissant et configurée de manière à engager l'élément d'arrêt disposé sur le bras.

2. Dispositif d'agrafe selon la revendication 1, dans lequel l'élément d'arrêt limite le déplacement distal de l'anneau coulissant.

3. Dispositif d'agrafe selon la revendication 1, dans lequel l'élément d'arrêt est dimensionné pour être au moins partiellement logé à l'intérieur de la dépression.

4. Dispositif d'agrafe selon la revendication 1, dans lequel l'élément d'arrêt comprend au moins un élément en forme de perle disposé sur le bras.

5. Dispositif d'agrafe selon la revendication 1, dans lequel ladite au moins une dépression engage de façon verrouillée l'élément d'arrêt qui est disposé sur le bras.

6. Dispositif d'agrafe selon la revendication 1, dans lequel le premier élément de retenue présente des régions proximale et distale, le dispositif d'agrafe comprenant en outre un deuxième élément de retenue présentant des régions proximale et distale, dans lequel le premier élément de retenue est configuré de manière à être couplé au deuxième élément de retenue avant le déploiement de l'agrafe, et dans lequel le premier élément de retenue est configuré de manière à être désengagé du deuxième élément de retenue après le déploiement de l'agrafe.

7. Dispositif d'agrafe selon la revendication 6, dans lequel le premier élément de retenue est configuré de manière à être désengagé du deuxième élément de retenue en rétractant de façon proximale le deuxième élément de retenue par rapport au premier élément de retenue.

8. Dispositif d'agrafe selon la revendication 7, dans lequel le premier élément de retenue et le deuxième élément de retenue présentent des forces magnétiques opposées, dans lequel l'application d'une force de retrait suffisante au deuxième élément de retenue entraîne le deuxième élément de retenue à se désengager du premier élément de retenue.

9. Dispositif d'agrafe selon la revendication 7, dans lequel le premier élément de retenue est couplé au deuxième élément de retenue en utilisant un agencement de roulement à billes.

10. Dispositif d'agrafe selon la revendication 6, comprenant en outre:
un fil s'étendant à partir de la région distale du deuxième élément de retenue et une bille attachée à une extrémité distale du fil; et
un canal et une douille formés dans la région proximale du premier élément de retenue, dans lequel le canal et la douille sont configurés de manière à recevoir le fil et la bille, respectivement, afin de coupler le premier élément de retenue au deuxième élément de retenue.

11. Dispositif d'agrafe selon la revendication 10, dans lequel l'anneau coulissant empêche le désengagement de la bille de la douille avant le déploiement de l'agrafe.

12. Dispositif d'agrafe selon la revendication 6, dans lequel le premier élément de retenue est désengagé du deuxième élément de retenue en provoquant la rotation du deuxième élément de retenue par rapport au premier élément de retenue.
